# DEMANDE DE BREVET EUROPEEN

(11) **EP 1 150 119 A1**
(43) Date de publication de la demande: **31.10.2001**
(21) Numéro de dépôt: 01400992.2
(22) Date de dépôt: 18.04.2001
(51) Int. Cl.: G01N 33/03, G01N 27/07, G01N 27/22, A47J 37/12

(54) **Capteur pour la mesure d'une caractéristique électrique d'un bain d'huile d'une friteuse industrielle**

(30) Priorité: 28.04.2000 FR 0005457
(71) Demandeur: CENTRE TECHNIQUE DES INDUSTRIES MECANIQUES, F-60300 Senlis (FR)
(72) Inventeur: Buisson, Raymond, 60300 Senlis (FR)
(74) Mandataire: Bertrand, Didier

(57) **Abrégé**

Ce capteur de mesure d'une caractéristique électrique d'un bain d'huile de friteuse, du type comportant deux plaques d'électrodes parallèles (5, 6), est caractérisé en ce que les plaques (5, 6) sont en acier inoxydable et sont séparées et assemblées par des espaceurs (7, 8) en céramique ou en polytétrafluorure d'éthylène.

## Description

La présente invention concerne un capteur pour la mesure d'une caractéristique d'un bain d'huile d'une friteuse industrielle.

Le document US 5 594 327 fait connaître un procédé de mesure de la conductivité de l'huile à l'aide d'un capteur de mesure constitué de deux électrodes plates en titane d'une très petite surface (2 cm²) séparées par un intervalle de 0,2 cm, le capteur étant destiné à mesurer la conductivité d'un échantillon d'huile prélevé dans le bain.

Le but de l'invention est de rendre cette mesure compatible avec une friteuse industrielle grâce à un capteur susceptible de rester à demeure dans le bain d'huile de la friteuse, et de concevoir un capteur compatible avec les exigences en matière de sécurité alimentaire, c'est-à-dire notamment offrant peu de prise à l'introduction et aux incrustations de déchets et de saletés.

Le but de l'invention est atteint grâce à un capteur de mesure d'une caractéristique électrique d'un bain d'huile de friteuse, du type comportant deux plaques d'électrodes parallèles, caractérisé en ce que les plaques sont en acier inoxydable et sont séparées et assemblées par des espaceurs en céramique ou en polytétrafluorure d'éthylène (Téflon® ). Ces plaques ont une surface importante, par exemple environ 30 cm x 6 cm et sont séparées par un intervalle de quelques millimètres, par exemple 8 mm.

Avantageusement, les espaceurs sont des barreaux de section rectangulaire, disposés par exemple aux extrémités des plaques et en leur milieu.

Avantageusement, les plaques d'électrode sont fixées aux espaceurs par des rivets aveugles. A cet effet, chaque rivet aveugle peut être fixé dans un trou de l'espaceur grâce à une rondelle arrière d'appui, notamment quand l'espaceur est en Téflon.

Avantageusement, chaque barreau espaceur comporte une zone de dilatation, réalisée par exemple sous forme de fentes parallèles alternées.

Avantageusement, les connexions électriques aux électrodes sont amenées par un tube soudé à une première électrode au niveau d'un trou traversant. Le tube enferme un câble connecté à l'autre électrode. Le câble et le tube sont séparés par un manchon isolant. Le manchon isolant est constitué par des enveloppes successives de gaine thermorétractable. Le manchon isolant incorpore les deux fils d'un thermocouple. Le câble traverse un espaceur.

Avantageusement, le tube et le câble sont reliés à une partie de mesure et de commande par une connexion comportant une pince fendue.

D'autres caractéristiques et avantages de la présente invention ressortiront de la description ci-après d'un exemple de réalisation de l'invention illustré dans les dessins annexés sur lesquels :

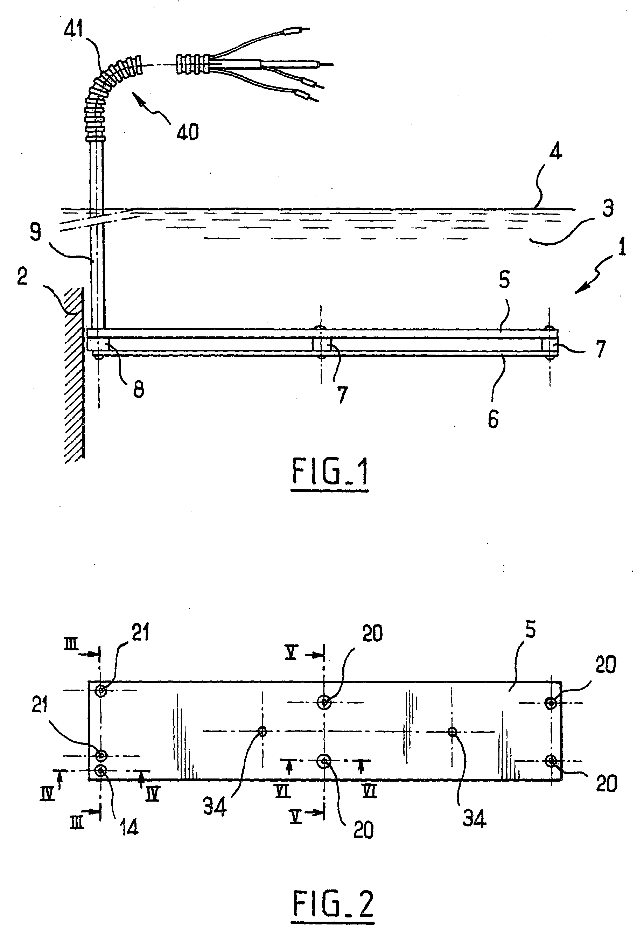

- la figure 1 est une vue de côté du capteur de l'invention plongé dans la cuve d'une friteuse,
- la figure 2 est une vue de dessus de l'électrode supérieure du capteur de la figure 1,
- la figure 3 est une coupe du capteur de la figure 1 au niveau de la ligne III-III de la figure 2,
- la figure 4 est une coupe du capteur de la figure 1 au niveau de la ligne IV-IV de la figure 2,
- la figure 5 est une coupe du capteur de la figure 1 au niveau de la ligne V-V de la figure 2,
- la figure 6 est une coupe du capteur de la figure 1 au niveau de la ligne VI-VI de la figure 2,
- la figure 7 est une vue de dessous d'un type d'espaceur entre deux plaques d'électrodes,
- la figure 8 est une coupe selon la ligne VIII-VIII de l'espaceur de la figure 7,
- la figure 9 est une vue de dessus de l'espaceur de la figure 7,
- la figure 10 représente le détail de la partie haute température de la connexion aux électrodes
- la figure 11 est une section XI-XI de la connexion de la figure 10,
- la figure 12 est une section XII-XII de la connexion de la figure 10,
- la figure 13 est une vue de détail en coupe d'une bague utilisée dans la connexion de la figure 10,
- la figure 14 est une vue de côté de certains des éléments intérieurs de la connexion de la figure 10, représentés sans la gaine thermorétractable ni la gaine inox.

Le capteur 1 de l'invention est plongé dans une cuve dont une partie de paroi 2 est représentée, remplie d'un bain d'huile 3 jusqu'à une surface 4.

Le capteur 1 se compose de deux électrodes planes rectangulaires supérieure 5 et inférieure 6 maintenant entre elles un intervalle constant grâce à des espaceurs 7 et 8.

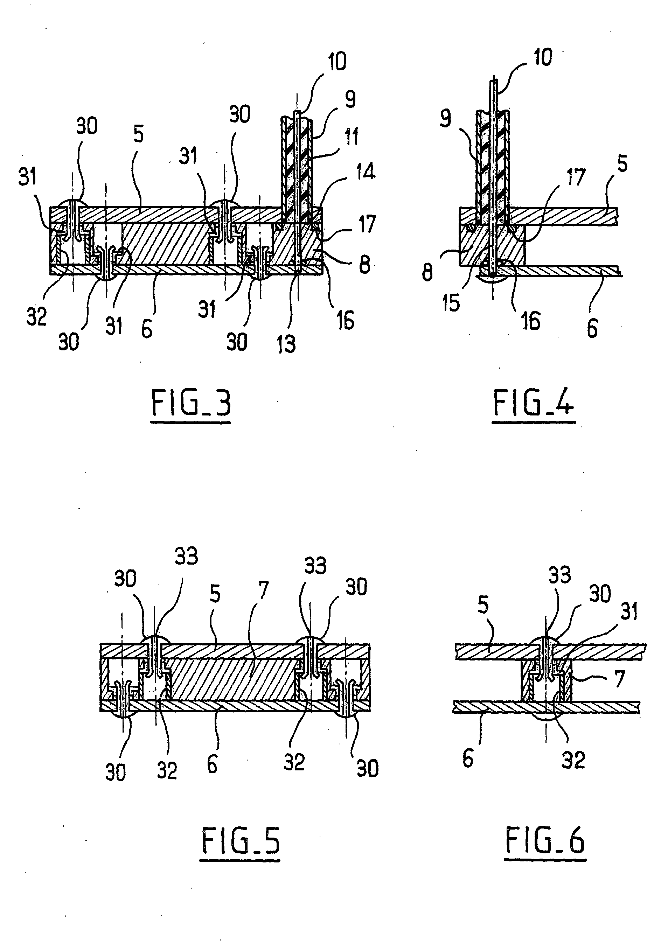

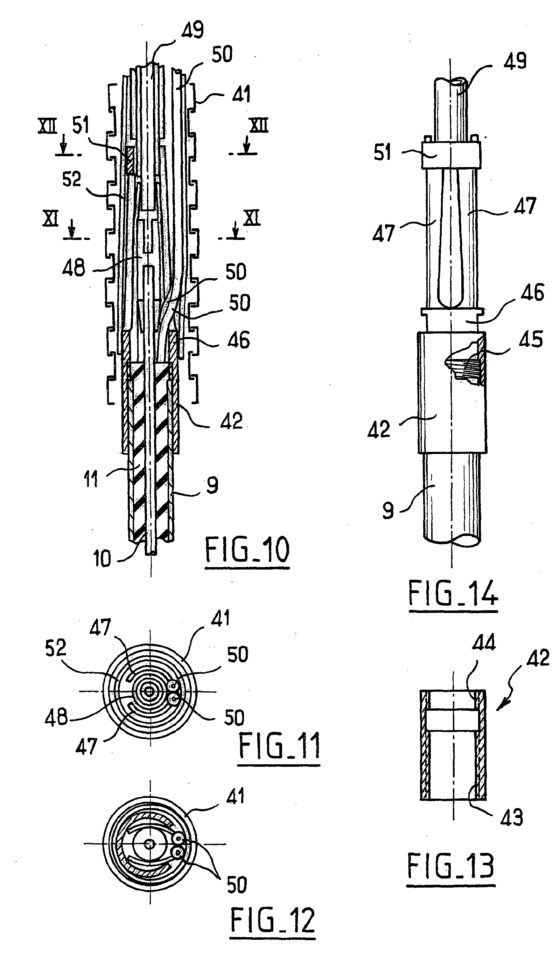

Un tube en inox 9 part vers le haut d'un coin de l'électrode supérieure 5 et renferme un câble coaxial 10 destiné à être raccordé à l'électrode inférieure 6, comme le montrent mieux les figures 3 et 4. Le câble 10 et le tube 9 sont isolés par un manchon 11 de caoutchouc silicone incorporant les fils de thermocouple (fils 50, cf. infra figures 10 à 12), le manchon étant formé par une succession de manchons thermorétractables enfilés les uns sur les autres. Le câble 10 est soudé à l'arc dans un trou 13 de l'électrode 6 et le tube 9 est soudé à l'arc dans un trou 14 de l'électrode supérieure 5. Le câble 10 passe aussi dans un trou traversant 15 prévu dans l'espaceur 8 et deux joints toriques 16, 17 en silicone assurent l'étanchéité entre l'espaceur 8 et les électrodes inférieure et supérieure au niveau du passage de câble 10 et du tube 9.

Les plaques d'électrodes 5 et 6 comportent des trous 20 et 21 permettant leur fixation par rivets avec interposition des deux espaceurs 7 et de l'espaceur 8, ce dernier ne différant des espaceurs 7 que par la position des passages de rivets décalée en raison du passage de câble 10.

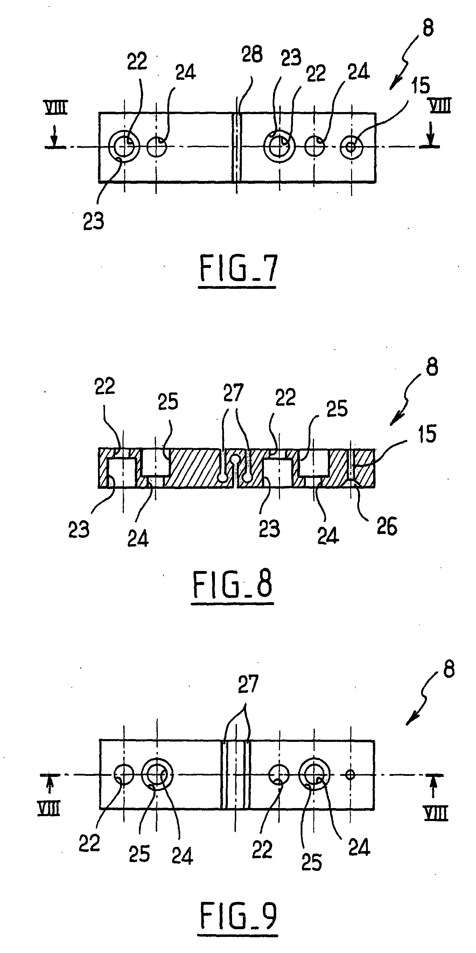

L'espaceur 8, visible plus en détail sur les figures 7 à 9, est un barreau de Téflon® ou de céramique de longueur correspondant à la largeur des plaques d'électrode, à section rectangulaire définissant l'écartement des électrodes et comportant un certain nombre de trous de fixation. Les trous supérieurs 22 débouchent sur une chambre cylindrique élargie 23 s'ouvrant à la partie inférieure, de même que les trous inférieurs 24 débouchent sur une chambre élargie 25 s'ouvrant à la partie supérieure. Le trou traversant 15 de passage de câble se termine à la partie inférieure par un fraisage conique 26 permettant de loger la soudure du câble 10. Pour permettre la dilatation du barreau espaceur 8, il est prévu vers son milieu trois fentes alternées parallèles, deux fentes 27 débouchant vers le haut encadrant une fente 28 débouchant vers le bas, les fentes ayant des parois parallèles et un fond arrondi élargi.

Les trous 20 et 21 des plaques et les trous 22 et 24 des espaceurs permettent de fixer l'ensemble grâce à la coopération de rivets aveugles 30 (figures 4 à 6) et de rondelles d'appui en inox 31 s'appuyant sur l'épaulement formé entre les trous 22, 24 et la chambre arrière 23, 25. La queue des rivets 30 vient s'épanouir à l'arrière desdites rondelles 31 après l'expansion des rivets. Les rondelles 31 destinées aux trous supérieurs 22 peuvent être maintenues en place avant rivetage par un manchon 32 placé dans la chambre arrière 23. Les rivets sont des rivets inox aveugles en chargeurs à broches perdues. Les trous axiaux 33 laissés dans les rivets 31 sont obturés après leur expansion par des bouchons en acier inoxydable, formés d'un court tronçon de fil d'inox.

La plaque d'électrode 5 peut aussi comporter des trous de fixation 34 pour fixer le capteur à un emplacement déterminé dans la cuve, par exemple au niveau de résistances de chauffage de l'huile.

La partie supérieure du tube 9 débouche au-dessus de la surface 4 du bain d'huile et est raccordée à un ensemble de mesure et de commande non représenté par une connexion souple 40 enfermée dans une gaine d'acier inoxydable spiralée ou annelée 41. Le détail de cette connexion au niveau de son raccord au tube 9 est représenté sur les figures 10 à 12. Sur l'extrémité supérieure du tube 9, dans lequel se trouvent le câble coaxial 10 et le manchon 11, se visse une bague d'inox 42 comportant un filetage intérieur inférieur avec pas à droite 43 (pour le tube 9) et un filetage intérieur supérieur avec pas à gauche 44. Dans ce filetage 44 vient se visser la partie inférieure filetée 45 d'une pièce cylindrique 46 portant deux extensions longitudinales 47 formant une pince fendue. Le câble 10 est soudé à son extrémité supérieure dans un embout à souder cylindrique 48, en cuivre ou en laiton, comportant deux trous borgnes opposés, permettant de le raccorder à un câble coaxial 49. La pince fendue enserre le câble 10, l'embout 48 et le câble coaxial 49, et est maintenue serré par un anneau fendu élastique 51. Les deux fils 50 de thermocouple qui sortent du manchon 11 passent entre les extensions 47 de la pince fendue et longent la pince fendue et son anneau fendu 51. Une gaine thermorétractable 52 est placée autour de cet ensemble avant de mettre en place la gaine inox 41.

## Revendications

1. Capteur de mesure d'une caractéristique électrique d'un bain d'huile de friteuse, du type comportant deux plaques d'électrodes parallèles (5, 6), **caractérisé en ce que** les plaques (5, 6) sont en acier inoxydable et sont séparées et assemblées par des espaceurs (7, 8) en céramique ou en polytétrafluorure d'éthylène, et **caractérisé en ce que** les connexions électriques aux électrodes sont amenées par un tube (9) soudé à une première électrode (5) au niveau d'un trou traversant (14)..

2. Capteur selon la revendication 1, **caractérisé en ce que** les espaceurs (7, 8) sont des barreaux de section rectangulaire.

3. Capteur selon la revendication 2, **caractérisé en ce que** les plaques d'électrode (5, 6) sont fixées aux espaceurs par des rivets aveugles (30).

4. Capteur selon la revendication 3, **caractérisé en ce que** chaque rivet aveugle est fixé dans un trou de l'espaceur grâce à une rondelle arrière d'appui.

5. Capteur selon l'une quelconque des revendications 2 à 4, **caractérisé en ce que** chaque barreau (7, 8) comporte une zone de dilatation.

6. Capteur selon la revendication 5, **caractérisé en ce que** le tube enferme un câble (10) connecté à l'autre électrode (6).

7. Capteur selon la revendication 6, **caractérisé en ce que** le câble (10) et le tube (9) sont séparés par un manchon isolant (11).

8. Capteur selon la revendication 7, **caractérisé en ce que** le manchon isolant (11) est constitué par des enveloppes successives de gaine thermorétractable.

9. Capteur selon l'une quelconque des revendications 7 ou 8, **caractérisé en ce que** le manchon isolant (11) incorpore les deux fils (50) d'un thermocouple.

10. Capteur selon l'une quelconque des revendications 6 à 9, **caractérisé en ce que** le câble (10) traverse un espaceur (8).

11. Capteur selon l'une quelconque des revendications 6 à 10, **caractérisé en ce que** le tube (9) et le câble (10) sont reliés à une partie de mesure et de commande par une connexion comportant une pince fendue (46, 47).
